# EUROPEAN PATENT SPECIFICATION

(11) **EP 2 511 288 B1**
(45) Date of publication and mention of the grant of the patent: **02.03.2016**
(21) Application number: 12163789.6
(22) Date of filing: 11.04.2012
(51) Int. Cl.: C07K 1/22, C07K 1/36, C07K 14/465, C07K 14/81

(54) **Method of obtaining inhibitors of cysteine peptidases with an electrophoretic purity from biological materials**
Verfahren zum Erhalten von Inhibitoren von Cystein-Peptidasen mit einer elektrophoretischen Reinheit aus biologischen Materialien
Procédé d'obtention d'inhibiteurs de cystéine-peptidases avec une pureté électrophorétique à partir de matières biologiques

(30) Priority: 11.04.2011 PL 39451411
(43) Date of publication of application: 17.10.2012
(73) Proprietor: Akademia Medyczna Im. Piastow Slaskich We Wroclawiu, 50-357 Wroclaw (PL)
(72) Inventor: Siewinski, Maciej, 53-134 Wroclaw (PL); Czarnecki, Jan, 58-508 Jelenia Góra (PL); Jujeczka, Stanislaw, 51-109 Wroclaw (PL); Seliga, Justyna, 59-220 Legnica (PL); Zuchowski, Aleksander, 55-050 Sobótka (PL)
(74) Representative: Witek, Rafal

(56) References cited:
- EP-A2- 0 188 262
- WO-A2-94/29348
- FONG D ET AL: "Bacterial expression of human cysteine proteinase inhibitor stefin A", FEBS LETTERS, ELSEVIER, AMSTERDAM, NL, vol. 257, no. 1, 23 October 1989 (1989-10-23), pages 55-58, XP025608143, ISSN: 0014-5793, DOI: 10.1016/0014-5793(89)81785-5 [retrieved on 1989-10-23]
- HUA-LI NIE ET AL: "Papain Immobilization on Chitosan-Coated Nylon Membrane: Preparation and Its Application in Cystatin Separation", BIOINFORMATICS AND BIOMEDICAL ENGINEERING , 2009. ICBBE 2009. 3RD INTERNATIONAL CONFERENCE ON, IEEE, PISCATAWAY, NJ, USA, 11 June 2009 (2009-06-11), pages 1-4, XP031488955, ISBN: 978-1-4244-2901-1
- SHANN-TZONG JIANG, SHINN-SHUENN TZENG, WUN-TSAI WU, GEN-HUNG CHEN: "Enhanced Expression of Chicken Cystatin as a Thioredoxin fusion Form in Escherichia coli AD494(DE3)pLysS and Its Effect on the Prevention of Surimi Gel Softening", JOURNAL OF AGRICULTURAL AND FOOD CHEMISTRY, vol. 50, 21 May 2002 (2002-05-21), pages 3731-3737, XP002678170,
- ANASTASI A ET AL: "CYSTATIN, A PROTEIN INHIBITOR OF CYSTEINE PROTEINASES IMPROVED PURIFICATION FROM EGG WHITE, CHARACTERIZATION, AND DETECTION IN CHICKEN SERUM", BIOCHEMICAL JOURNAL, THE BIOCHEMICAL SOCIETY, LONDON, GB, vol. 211, no. 1, 1 January 1983 (1983-01-01), pages 129-138, XP009023121, ISSN: 0264-6021

## Description

The subject of the present invention is a method of producing inhibitors of cysteine peptidases of electrophoretic purity from biological materials, which regulate the activity of enzymes that play a strategic role in the development of many diseases, mainly oncogenic processes such as transformation, invasion, metastasis or angiogenesis as well as diseases connected with viral, bacterial, fungal or protozoan infections that induce such diseases as AIDS or malaria, and which lead to irreversible changes in developing osteoporosis, Alzheimer's disease, dystrophy, myopathy or paradontosis (Keppler D. 2006; Hugli T.E. 1996). The present invention is useful in medicine.

The role of cathepsins, cysteine peptidases, is known in the abovementioned diseases. Moreover, the potential possibilities of using their specific inhibitors, mainly synthetic ones, in regulating the activity of these enzymes were defined. The possibility of using inhibitors of these enzymes as potential drug ingredients are known. It is suggested that such drugs may be useful in the control of the development of those diseases, in which a large role is played by enzymes with cysteine in their active centre. Such synthetically obtained inhibitors exhibited the characteristics of inhibitors of diseases mainly modulated by cathepsins B, K, L *in vitro*, but were, unfortunately, toxic *in vivo*, and thus could not be used in therapy. The results obtained using synthetic inhibitors and expensive peptide analogues obtained in small quantities have, however, made it possible to note the important fact of their therapeutic effectiveness *in vitro* and *in vivo* in animal models. It was also confirmed that the direction of research into new cysteine peptidase inhibitor drugs may yield interesting therapeutic effects, if the problem of their toxicity may be solved (Grub A. et al. 1991; Zimmerman MP. et al. 1999; Obala R. et al. 2010).

Currently, intensive research is conducted with regard to the isolation of peptide inhibitors of cysteine peptidases from biological material, in quantities sufficient for industrial use. Such a method should be efficient enough that peptide inhibitors from natural sources will be equally useful as synthetic ones, but due to their properties will not be toxic or immunogenic to the patient. A novel therapy can be designed around them, which may be termed "inhibitor therapy". From the state of the art it is known that one source of inhibitors of cysteine peptidases is egg white protein. It is produced based on American patent US4902509 (Turk V., Brzin J. 1990) using immobilised carboxymethylpapain, bound to a known carrier, using affinity chromatography. The efficiency of this method is, however, small when considering the quantity of cysteine peptidase inhibitors in the raw material. Additionally, there is the very high cost of reagents and purification methods used, entailed by the need to use double crystallised papain prior to its carboxymethylation, which amounts to about 500 euro per 1 mg of pure inhibitor. This limits the use of inhibitors thus purified as potential drug ingredients. Polish patent PL288769 discloses a method of producing inhibitors of thiol proteases from the urine of malignant tumour patients using affinity chromatography using papain or phycin as biosorbents immobilised on a known carrier. Urine that has been initially purified of mineral contents is loaded onto a chromatography column at least twice, eluted with a pH 6.5 buffer with 0.2 M sodium chloride. The biosorbent is then unpacked into water, the pH is adjusted to 1 to 4 and heated to 60°C to 100°C, cooled to room temperature, neutralised, the precipitate is centrifuged off and the supernatant is condensed and stored at -20°C (Siewiński 1991). Polish patent PL174636 discloses a method of producing cysteine peptidase inhibitors from egg protein using affinity chromatography and papain or phycin as biosorbents immobilised on a known carrier, wherein the supernatant is loaded onto a chromatography column at least twice and is eluted with a buffer with a pH of about 6.0 and 0.2 M sodium chloride. The proteins are added slowly, in small doses to phosphate buffer containing ethylenediaminotetraacetic acid and cysteine, resulting in pH = 6, whereas the biosorbent from the column is suspended in water and the pH is adjusted to 1-4 or 9-12, whereafter it is first heated to a temperature above 60°C, then cooled, neutralised, centrifuged at 30000 RPM for 20 minutes, and the precipitate is separated off. The supernatant is condensed, purified of sodium chloride on membranes and of microorganisms on bacterial filters and packed into ampoules (Siewinski et al., 1998). This is a similar procedure to that used for inhibitors obtained from the amniotic fluid of human and animal placentae, which are useful in the inhibition of infective and neoplasmic diseases, as described in Polish patent 334758. Methods of producing inhibitors disclosed in Polish patents PL288769, PL174636 and application PL334758 make it possible to obtain raw preparations. Prior to use they require further purification, in several stages, using expensive and inefficient protein purification methods. Another problem with the above described methods is that it is not possible to reuse the column bed, which additionally increases costs. To summarise, the use of known and used methods of purifying inhibitor preparations from known sources is costly, complicated and inefficient, because only small quantities of cysteine peptidase inhibitors with low activity are obtained from the resulting eluate, and the methods used require costly procedures and reagents. No electrophoretically pure preparations were obtained during the first stage of purification of cysteine peptidase inhibitors, neither using carboxymethylpapain, as in the American patent, nor papain according to the methods indicated in the Polish patents, where temperature and pH shifts were used. Thus, there was a need for further purification steps so as to reach preparation purity, which causes complications and decreases efficiency. This also necessitated further expenditures, which increased the costs of the inhibitor produced, and for this reason its utility in the pharmaceutical industry was limited. Despite over 20 years of attempts made to obtain cysteine peptidase inhibitors from various sources, the superficial simplicity and numerous techniques which can be used, thus far there has been no method of producing the inhibitors on a technical scale. It has become necessary to design a method, which would efficiently and effectively facilitate the production of large quantities of cysteine peptidase inhibitors of electrophoretic purity and a high activity, from natural sources, and which would facilitate the re-use of the chromatography gel using low-energy reclamation methods. Unexpectedly, this problem was solved by the present invention.

The subject of the present invention is a method of producing cysteine peptidase inhibitors of electrophoretic purity from biological materials encompassing the preparation of a protein mass for producing inhibitors of cysteine peptidases, after which the cysteine peptidase inhibitors are extracted and then the chromatography gel for extracting cysteine peptidase inhibitors is reclaimed, characterised in that it encompasses:
a) preparation of a protein mass for the production of cysteine peptidase inhibitors by treating the protein mass with an acidic pH close to 2, as well as a high temperature, close to 90°C for 10 to 20 minutes, whereafter the solution is frozen for several hours, preferably 12;
b) production of cysteine peptidase inhibitors by at least one affinity chromatography, preferably in a column, against carriers bound to papain or phycin or another derivative of plant cysteine peptidases from the prepared, defrosted protein mass of stage a) to an activity of at least 5 IU/mg of eluent following the first elution, wherein the reclamation of the inhibitors bound to the peptide is performed using a solution of 20% glycerol, with a pH from 9.5 to 12 at a temperature from 5°C to 55°C with multiple reuse of the bed, or at a temperature from 60°C to 100°C with a single use. Equally preferably, a method according to the present invention is characterised in that an at least twofold elution occurs on the same chromatography gel of stage b). In the next preferable embodiment of the present invention, the method is characterised in that the protein mass is obtained from eggs diluted with distilled water, preferably 1:1 egg mass to water or another biological material selected from a group containing plant homogenates such as potatoes, soybeans, rice, mistletoe or mammalian tissues such as placentae, cancerous tissues following surgical removal as well as amniotic fluid, urine and oedemic fluid. Equally preferably, a method according to the present invention is characterised in that the production of cysteine peptidase inhibitors in stage b) encompasses the centrifugation of the defrosted protein mass of stage a), preferably over 20 minutes at 30000 RPM. In the next preferable embodiment of the present invention, the method is characterised in that in stage b) the centrifugation supernatant is loaded onto a column, preferably packed with Sepharose 4B bound to a peptide, at least twice, preferably with 0.05% sodium sorbate and then the column is unpacked and washed, preferably first in 0.2 M NaCl as well as distilled water, until biological protein disappears from the eluate. Equally preferably, a method according to the present invention is characterised in that the yielded eluate following the final wash in stage b) is acidified, preferably to pH 2.0 and heated, preferably to 90°C for 10-20 minutes, chilled and neutralised. Preferably a method according to the present invention is characterised in that the elution of stage b) is performed until electrophoretic purity, preferably two, three or 4 times on consecutive chromatography gels with immobilised peptides and an activity of at least 5 IU/mg eluent after the first elution is reached, wherein preferably the elutions are conducted at pH 11 at room temperature. Equally preferably a method according to the present invention is characterised in that in stage b) following the final elution the chromatography gel the immobilised enzyme is reactivated through incubation in buffer, preferably over minimum 12 hours at a temperature of 4°C in phosphate buffer, pH 6.5 and 2 mM dithiothreitol, 2 mM ethylenediaminotetraacetic acid (EDTA) and 0.05 % mercaptoethanol, in order to reactivate the enzyme.

The subject of the present invention is a technology which makes it possible to produce cysteine peptidase inhibitors in industrial quantities at prices decidedly lower than using the abovementioned laboratory methods. Furthermore, the resulting preparation does not require further purification using other techniques, in contrast to inhibitors produced at laboratory scale and may be used directly. For example, the method is based on repeated loading of a biological preparation containing active inhibitors of cysteine peptidases onto a column packed with a known chromatography gel bound with papain, phycin or another cysteine peptidase as defined in the claims, unpacking the chromatography gel, which is then washed on a Buchner funnel with 0.2 M NaCl and water until there is no protein in the filtrate, whereafter the chromatography gel is reloaded onto the same column. The reclamation of inhibitors bound to an enzyme combined with the aforementioned enzymes is based on their elution from the chromatography gel a solution containing 20 % glycerol to pH from 9.5 to 12 at a temperature range from 8 to 55°C. The collected eluate is first neutralised, and its activity and electrophoretic purity are determined after desalting and concentration on a membrane porous to molecules of up to 5 kDa while maintaining the 20 % glycerol concentration. If needed, if the inhibitor activity is too low, the solution is acidified, heated to 90°C for 5 minutes, cooled to room temperature, neutralised and phosphate buffer with a pH of 6.5 is added, maintaining the 20 % glycerol concentration, it is concentrated and desalted on a membrane permeable to molecules up to 5 kDa. When the preparation fails to meet purity requirements, it is once again loaded onto a new column packed with the same chromatography gel bound to the same enzymes, and membrane on which the inhibitor is again washed using the abovementioned method. This purification is performed 2-4 times on a sequence of columns packed with a known chromatography gel with immobilised papain, phycin or another cysteine peptidase as defined in the claims until such a time as the preparation is electrophoretically pure and exhibits the desired activity per mg protein. Every time the inhibitor is loaded onto the column, the chromatography gel is unpacked, washed of unbound substances, whereafter the column is repacked with it. The inhibitor is eluted from the column using a solution with a pH from 9.5 to 12 at a temperature range from 8°C to 55°C. The given example embodiment of a method according to the present invention makes it possible to utilise a chromatography gel numerous times. It is significant that prior to each loading, a solution containing the isolated inhibitor is incubated on a chromatography gel with the immobilised enzyme over a minimum 12 hour period at a temperature of 4°C in phosphate buffer with a pH of 6.5 and 2 mM dithiothreitol, 2 mM ethylenediaminotetraaceticacid (EDTA) and 0.05 % mercaptoethanol in order to reactivate the enzyme. There is another example method of obtaining inhibitors using the chromatography a single time. It is based on isolating the inhibitor from the chromatography gel at a temperature of 60°C to 100°C at a pH of 1 to 6 or 8 to 12, cooling the whole system, neutralising, freezing for 24 hours, defrosting, adding glycerol to 20 %, desalting on a membrane permeable up to 5 kDa, passing through antibacterial filters and storing in ampoules or another sterile form at a temperature of 4°C or frozen. The present invention relates to a method of producing cysteine peptidase inhibitors from egg proteins as well as placentae and amniotic fluids both of animals and humans, homogenates of cancerous tissues removed surgically, from plants such as potatoes, soybeans, rice and mistletoe using affinity chromatography on a column packed with known carriers bound to papain, phycin or another cysteine peptidase as defined in the claims, through repeated loading of the supernatant onto the chromatography column, and then washing out unbound molecules from the chromatography gel, eluting the from cysteine peptidase inhibitors the column using a solution with a pH from 9.5 to 12 at a temperature range from 8°C to 55°C with multiple usage of the chromatography gel or from 60°C to 100°C with single usage of the chromatography gel, cooling to room temperature and neutralisation, an then centrifugation and, after passing through antibacterial filters, using the supernatants for further purposes. Inhibitors of cysteine peptidases are protected against dimerisation by 20 % glycerol. An example embodiment of the present invention is based on the fact that the eluate is manufactured from homogenates of various source substances through centrifugation, pH selection from 9.5 to 12, heating from 8°C to 55°C or from 60°C to 100°C, and then cooling to room temperature, neutralising, centrifugation and freezing at a temperature of -20°C for a time of preferably 24 hours, whereafter it is defrosted and centrifuged. The supernatant is used, loaded a number of times onto a column packed with a known chromatography gel bound to papain, phycin or another cysteine peptidase as defined in the claims. After obtaining the inhibitor-enzyme complex, the chromatography gel is unpacked, washed with 0.2 M NaCl and water until no protein is found in the filtrate, the chromatography gel repacked in column. The inhibitor is washed out of the column at a pH from 9.5 to 12, at a temperature range from 8°C to 55°C wherein the chromatography is used numerous times or heating the chromatography gel unpacked from the column in the same pH range to a temperature from 60°C to 100°C, when the chromatography gel is used a single time. The obtained inhibitors are stored in 20 % glycerol, to secure them against dimerisation. When the inhibitors lack the required purity, the purification is repeated on another column packed with the same chromatography gel. The resulting solution is desalted on membranes permeable to molecules of up to 5 kDa. In the same fashion, an aqueous solution of cysteine peptidase inhibitors can be concentrated to its desired density and volume. The activity is determined colorimetrically or spectrfluorometrically against papain or cathepsin B. Finally, the inhibitors in sterile form, following purification on a microbiological filter, are stored in ampoules at 4°C or frozen in sterile plastic containers. Solutions containing electrophoretically pure inhibitors of cysteine peptidases are purified of sodium chloride using a filtration device using selected membranes, and this method is used for concentrating the inhibitor solution. An example method of producing cysteine peptidase inhibitors according to the present invention is based on the repetition of the described method of affinity chromatography, until the production of electrophoretically pure fractions. This method makes it possible to obtain cysteine peptidase inhibitors from various sources with an efficiency in excess of 50% of their initial activity, using a cheaper, more efficient method than in earlier methods of obtaining them. The glycerol present in the preparation is not an obstacle to use in injections or intravenous infusions, nor to external use such as in the treatment of paradontosis or mycoses.

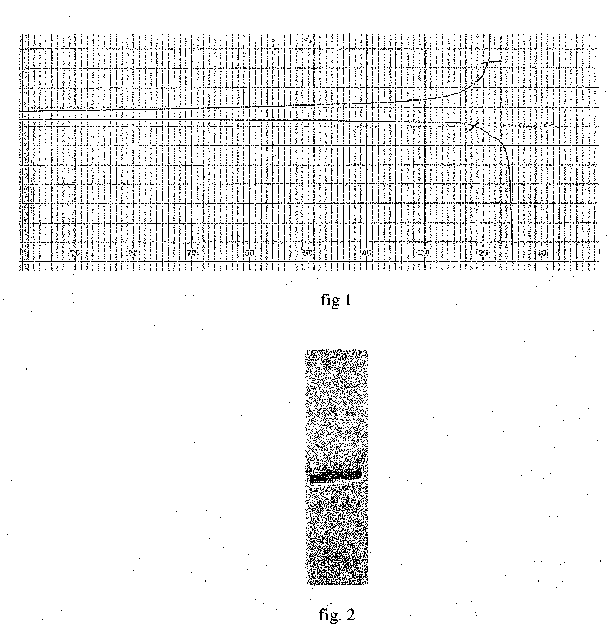

The attached figures illustrate the example embodiments of the present invention, wherein Fig. 1 represents a chromatogram of the cysteine peptidase inhibitors yielded during purification of egg proteins and elution with a buffer at pH 11 and room temperature, absorbance on a fraction collector at 280 nm wavelength, whereas Fig. 2 shows the result of electrophoresis on a 12% PAGE gel, of the cysteine peptidase inhibitor preparation yielded from egg proteins purified using elution with a buffer at a pH of 11 at room temperature, wherein 3 µg protein were loaded onto the gel.

### Example 1. Elution at room temperature using a buffer with a pH of 9.5.

3 kg of protein mass is diluted with 3 litres of distilled water, and then acidified to pH 2.0, heated to 90°C for 10-20 minutes, whereafter it is cooled to room temperature, neutralised and frozen for at least 12 hours. Next, it is defrosted, centrifuged for 20 minutes at 30000 RPM and the supernatant with 0.05 % sodium sorbate is loaded 3 -5 times onto a 5000 cm³ column packed with Sepharose 4B bound to papain. After the final loading, the column is unpacked and washed on a Buchner funnel first in 0.2 M NaCl as well as distilled water until no protein is observed in the filtrate. The column is packed with the washed chromatography gel and the inhibitor bound to it is eluted with 0.01 M NaHCO₃ with 20 % glycerol and a pH of 11.0 with an addition of 0.05 % sodium sorbate, collecting the protein-containing fractions, which when pooled have a volume of about 2 litres. The preparation is the acidified to pH 2.0, heated to 90°C for 10-20 minutes, whereafter it is cooled to room temperature and neutralised, whereafter protein content is determined. On average, the yield is 1 mg protein/ml with an activity of 2.0 to 3.5 IU/ml (2- 3.5 IU/mg protein).

The solution yielded from the purification on the first column is loaded onto a column of 400 cm³ packed with Sepharose 4B-papain 3-5 times, the chromatography gel is unpacked and washed with 0.2 M NaCl as well as distilled water until no protein is observed in the filtrate. Next, the column is packed with the washed chromatography gel and the inhibitor bound to it is eluted at room temperature using carbonate buffer with a pH of 9.5 with an addition of 20 % glycerol, collecting the protein-containing fractions. This yields 400 ml of eluate (with 20 % glycerol), which is acidified to pH 2.0, heated to 90°C for 10-20 minutes, cooled, neutralised and inhibitor activity is determined. This yields a preparation with an activity of 6.5 IU/ml, containing about 30 mg protein with an activity of 86.6 IU/mg.

### Example 2. Elution at room temperature using a buffer with a pH of 11.

3 kg of protein mass is diluted with 3 litres of distilled water, and then acidified to pH 2.0, heated to 90°C for 10-20 minutes, whereafter it is cooled to room temperature, neutralised and frozen for at least 12 hours. Next, it is defrosted, centrifuged for 20 minutes at 30000 RPM and the supernatant with 0.05 % sodium sorbate is loaded 3 -5 times onto a 5000 cm³ column packed with Sepharose 4B bound to papain. After the final loading, the column is unpacked and washed on a Buchner funnel first in 0.2 M NaCl as well as distilled water until no protein is observed in the filtrate. The column is packed with the washed chromatography gel and the inhibitor bound to it is eluted with 0.01 M NaHCO₃ with 20 % glycerol and a pH of 11 ,O with an addition of 0.05 % sodium sorbate, collecting the protein-containing fractions, which when pooled have a volume of about 2 litres. The preparation is the acidified to pH 2.0, heated to 90°C for 10-20 minutes, whereafter it is cooled to room temperature and neutralised, whereafter protein content is determined. On average, the yield is 1 mg protein/ml with an activity of 2.0 to 3.5 IU/ml (2- 3.5 IU/mg protein).

The solution yielded from the purification on the first column is loaded onto a column of 400 cm³ packed with Sepharose 4B-papain 3-5 times, the chromatography gel is unpacked and washed with 0.2 M NaCl as well as distilled water until no protein is observed in the filtrate. Next, the column is packed with the washed chromatography gel and the inhibitor bound to it is eluted at room temperature using carbonate buffer with a pH of 11 with an addition of 20 % glycerol, collecting the protein-containing fractions. This yields 400 ml of eluate (with 20 % glycerol), which is acidified to pH 2.0, heated to 90°C for 10-20 minutes, cooled, neutralised and inhibitor activity is determined. This yields a preparation with an activity of 15-18 IU/ml, containing about 60 mg protein with an activity of 100-120 IU/mg.

### Example 3. Elution at room temperature using a buffer with a pH of 12.

3 kg of protein mass is diluted with 3 litres of distilled water, and then acidified to pH 2.0, heated to 90°C for 10-20 minutes, whereafter it is cooled to room temperature, neutralised and frozen for at least 12 hours. Next, it is defrosted, centrifuged for 20 minutes at 30000 RPM and the supernatant with 0.05 % sodium sorbate is loaded 3 -5 times onto a 5000 cm³ column packed with Sepharose 4B bound to papain. After the final loading, the column is unpacked and washed on a Buchner funnel first in 0.2 M NaCl as well as distilled water until no protein is observed in the filtrate. The column is packed with the washed chromatography gel and the inhibitor bound to it is eluted with 0.01 M NaHCO₃ with 20 % glycerol and a pH of 11 ,O with an addition of 0.05 % sodium sorbate, collecting the protein-containing fractions, which when pooled have a volume of about 2 litres. The preparation is the acidified to pH 2.0, heated to 90°C for 10-20 minutes, whereafter it is cooled to room temperature and neutralised, whereafter protein content is determined. On average, the yield is 1 mg protein/ml with an activity of 2.0 to 3.5 IU/ml (2- 3.5 IU/mg protein).

The solution yielded from the purification on the first column is loaded onto a column of 500 cm³ packed with Sepharose 4B-papain 3-5 times, the chromatography gel is unpacked and washed with 0.2 M NaCl as well as distilled water until no protein is observed in the filtrate. Next, the column is packed with the washed chromatography gel and the inhibitor bound to it is eluted at room temperature using carbonate buffer with a pH of 12 with an addition of 15 % glycerol, collecting the protein-containing fractions. This yields 160 ml of eluate (z 15 % glycerol), which is acidified to pH 2.0, heated to 90°C for 10-20 minutes, cooled, neutralised and inhibitor activity is determined. This yields a preparation with an activity of 17,9 IU/ml, containing about 20.8 mg protein with an activity of 137,7 IU/mg.

### Example 4. Additional purification on a third column.

100 ml of preparation obtained according to the method described in Example 1 were loaded onto a 100 cm³ column with Sepharose 4B-papain three times, the column was rinsed with 2000 cm³ of distilled water, an then the inhibitor was eluted with 0.01 M NaHC0₃ with 20 % glycerol, collecting the protein-containing fractions. This yielded 80 ml a solution, which was brought to pH 2.0, heated to 90°C for 10-20 minutes, whereafter it was cooled to room temperature and neutralised, yielding a preparation with an activity of 15.6 IU/ml, a protein concentration of 0.036 mg/ml and with an activity of 433.3 IU/mg.

### Example 5. Elution at a temperature of 8°C using a buffer with a pH of 11.

3 kg of protein mass is diluted with 3 litres of distilled water, and then acidified to pH 2.0, heated to 90°C for 10-20 minutes, whereafter it is cooled to room temperature, neutralised and frozen for at least 12 hours. Next, it is defrosted, centrifuged for 20 minutes at 30000 RPM and the supernatant with 0.05 % sodium sorbate is loaded 3 -5 times onto a 5000 cm³ column packed with Sepharose 4B bound to papain. After the final loading, the column is unpacked and washed on a Buchner funnel first in 0.2 M NaCl as well as distilled water until no protein is observed in the filtrate. The column is packed with the washed chromatography gel and the inhibitor bound to it is eluted with 0.01 M NaHC0₃ with 20 % glycerol and a pH of 11.0 with an addition of 0.05 % sodium sorbate, collecting the protein-containing fractions, which when pooled have a volume of about 2 litres. The preparation is the acidified to pH 2.0, heated to 90°C for 10-20 minutes, whereafter it is cooled to room temperature and neutralised, whereafter protein content is determined. On average, the yield is 1 mg protein/ml with an activity of 2.0 to 3.5 IU/ml (2- 3.5 IU/mg protein).

Half of the preparation obtained after the purification on the first column is loaded onto a column of 200 cm³ with a heating jacket, packed with Sepharose 4B-papain 3-5 times, the chromatography gel is unpacked and washed with 0.2 M NaCl as well as distilled water until no protein is observed in the filtrate. Next, the column is packed with the washed chromatography gel, cooled to 8°C and the inhibitor bound to it is eluted using carbonate buffer with a pH of 11 at a temperature of 8°C with an addition of 20 % glycerol, collecting the protein-containing fractions. This yields 200 ml of eluate (with 20 % glycerol), which is acidified to pH 2.0, heated to 90°C for 10-20 minutes, cooled, neutralised and inhibitor activity is determined. This yields a preparation with an activity of 2.09 IU/ml, containing about 4,8 mg protein with an activity of 87.08 IU/mg.

### Example 6. Elution at a temperature of 55°C using a buffer with a pH of 11.

3 kg of protein mass is diluted with 3 litres of distilled water, and then acidified to pH 2.0, heated to 90°C for 10-20 minutes, whereafter it is cooled to room temperature, neutralised and frozen for at least 12 hours. Next, it is defrosted, centrifuged for 20 minutes at 30000 RPM and the supernatant with 0.05 % sodium sorbate is loaded 3 -5 times onto a 5000 cm³ column packed with Sepharose 4B bound to papain. After the final loading, the column is unpacked and washed on a Buchner funnel first in 0.2 M NaCl as well as distilled water until no protein is observed in the filtrate. The column is packed with the washed chromatography gel and the inhibitor bound to it is eluted with 0.01 M NaHC0₃ with 20 % glycerol and a pH of 11 ,O with an addition of 0.05 % sodium sorbate, collecting the protein-containing fractions, which when pooled have a volume of about 2 litres. The preparation is the acidified to pH 2.0, heated to 90°C for 10-20 minutes, whereafter it is cooled to room temperature and neutralised, whereafter protein content is determined. On average, the yield is 1 mg protein/ml with an activity of 2.0 to 3.5 IU/ml (2- 3.5 IU/mg protein).

Half of the preparation obtained after the purification on the first column is loaded onto a column of 200 cm³ with a heating jacket packed with Sepharose 4B-papain 3-5 times, the chromatography gel is unpacked and washed with 0.2 M NaCl as well as distilled water until no protein is observed in the filtrate. Next, the column is packed with the washed chromatography gel, heated to 55°C and the inhibitor bound to it is eluted using carbonate buffer with a pH of 11 at a temperature of 55°C with an addition of 20 % glycerol, collecting the protein-containing fractions. This yields 200 ml of eluate (with 20 % glycerol), which is acidified to pH 2.0, heated to 90°C for 1020 minutes, cooled, neutralised and inhibitor activity is determined. This yields a preparation with an activity of 1,15 IU/ml, containing about 38 mg protein with an activity of 6.06 IU/mg.

### Example 7. Purification with boiling of the chromatography gel.

3 kg of the chicken egg protein mass were initially purified through acidification to pH 2.0 and heating to 90°C, cooling and neutralisation. Next, the entirety was frozen for 12 hours, centrifuged at 4000 RPM for 20 minutes collecting the supernatant. The supernatant was loaded onto a column of 500 cm³ with Sepharose 4B-papain at a rate of 2 ml/min. The gel was rinsed free of protein, transferred to a flask, acidified to pH 2.0 and heated to 90°C. Next, it was cooled, neutralised, frozen and centrifuged. This yielded 640 ml a solution containing 1 mg protein/ml with an activity of 4,5 IU/mg protein.

### Example 8. Obtaining cysteine peptidase inhibitors from potatoes.

3.20 kg clean potatoes were sliced, an then mixed with 3.2 litres of distilled water, acidified to pH 2.0, heated to 90°C for 20 minutes. Next, it was cooled, neutralised and centrifuged for 20 minutes at 30 000 RPM collecting the supernatant, which was frozen for 24 hours, whereafter it was defrosted and again centrifuged for 20 minutes at 30 000 RPM as well as filtered through filter tissue. A slightly opalescent solution of 1.8L litres was loaded twice onto a 150 ml column packed with Sepharose 4B-papain. The gel was unpacked and then rinsed with water on a Buchner funnel. The gel was again loaded onto a column and the protein was eluted out at room temperature using a carbonate buffer, 0.01 M NaHC0₃, pH 11, with an addition of 20 % glycerol, collecting the protein-containing fractions. 100 ml eluate was collected. The entirety was neutralised and the protein content was determined as well as the activity of cysteine peptidase against papain using the BANA assay. This yields a preparation with an activity of 3.08 IU/ml, containing about 10 mg protein with an activity of 30.8 IU/mg.

### Example 9. Activity assay

The activity of cysteine peptidase inhibitors was determined in relation to BANA according to the method described in Siewiński M. (1991): Method of purification of thiol proteinase inhibitors from human urine. Cancer Biochem. Biophys. 12,33-43. The table compiles the properties of preparations yielded from 3 kg of protein mass (*in relation to 3 kg of protein mass) with elution with buffers at various temperatures and pH levels. The last column gives the yielded inhibitor units. The volume may be modified over concentration on appropriate membranes. For room temperature and pH 11, we give an average range of obtained parameters. This variant of the preparation was repeated several times. The details were described in earlier example embodiments.

| Preparation yielded by using the indicated temperature/pH | Protein content | Preparation activity | Units yielded |
|---|---|---|---|
| room temperature pH 9.5 | 75 µg/ml | 86.6 IU/mg | 2600 IU |
| room temperature; pH 11 | 150 µg/ml | 100-120 IU/mg | 6000-7200 IU |
| room temperature; pH 12 | 130 µg/ml | 137,7 IU/mg | 2864 IU |
| temperature 8°C/pH 11 | 24 µg/ml | 87,1 IU/mg | 836 IU * |
| temperature 55°CI pH 11 | 190 µg/ml | 6.05 IU/mg | 460 IU * |
| purification with boiling of the chromatography gel | 1 mg/ml | 4,5 IU/mg | 2880 IU |

## Claims

1. A method of producing cysteine peptidase inhibitors of electrophoretic purity from biological materials encompassing the preparation of a protein mass to yield cysteine peptidase inhibitors, after which the production of cysteine peptidase inhibitors occurs and then the reclamation of the chromatography gel to yield cysteine peptidase inhibitors, **characterised in that** it encompasses:
a) the preparation of a protein mass to yield cysteine peptidase inhibitors by acting on a protein mass solution with an acidic pH close to pH 2, as well as a high temperature close to 90°C for 10 to 20 minutes, after which the protein mass solution is frozen for several hours, preferably 12;
b) the production of cysteine peptidase inhibitors through at least one affinity chromatography, preferably on a column, against carriers with bound papain or phycin or another derivative of plant cysteine peptidases using defrosted protein mass of stage a) with an activity of at least 5 IU/mg in the eluent following the first elution, wherein the reclamation of inhibitors bound to the peptide is conducted with 20% glycerol, with a pH of from 9.5 to 12 at a temperature from 8°C to 55°C during multiple usage of the chromatography gel, or at a temperature of from 60°C to 100°C with single usage of the chromatography gel.

2. A method according to Claim 1, **characterised in that** an at least twofold elution is conducted on the same chromatography gel of stage b).

3. A method according to Claim 1 or 2, **characterised in that** the protein mass is obtained from eggs diluted with distilled water, preferably 1:1 of the egg mass to water or another biological material selected from a group containing plant homogenates such as potatoes, soybeans, rice, mistletoe or mammalian tissues such as placentae, cancerous tissues removed during surgery as well as amniotic fluid, urine and oedemic fluid.

4. A method according to Claim 1, **characterised in that** the production of cysteine peptidase inhibitors in stage b) encompasses centrifugation of the defrosted protein mass of stage a), preferably over 20 minutes at 30 000 RPM.

5. A method according to Claim 4, **characterised in that** in stage b) centrifuged supernatant is loaded onto a column, preferably packed with Sepharose 4B bound to a cysteine peptidase, at least twice, preferably with 0.05 % sodium sorbate and then washed, preferably first in 0.2 M NaCl and then in distilled water until no biological protein is found in the filtrate.

6. A method according to Claim 1, **characterised in that** the yielded eluate following the final elution in stage b) is acidified, preferably to pH 2.0 and heated, preferably to 90°C for 10-20 minutes, cooled and neutralised.

7. A method according to Claim 1, **characterised in that** elutions of stage b) are conducted until the point of electrophoretic purity, preferably two, three or 4 times onto subsequent chromatography gel columns with immobilised cysteine peptidase yielding an activity of at least 5 IU/mg in the eluent following the first elution from the first affinity chromatography, wherein preferably the elutions are conducted at pH 11 and room temperature.

8. A method according to Claim 1, **characterised in that** in stage b) following the elution, the chromatography gel with the immobilised enzyme is reactivated through incubation in a buffer, preferably over a minimum of 12 hours at 4°C in phosphate buffer with a pH of 6.5 and 2 mM dithiothreitol, 2 mM ethylenediaminotetraaceticacid (EDTA) and 0.05 % mercaptoethanol, in order to reactivate the enzyme.

## Patentansprüche

1. Verfahren zur Herstellung von Cystein-Peptidase-Inhibitoren von elektrophoretischer Reinheit aus biologischem Material, umfassend die Bereitstellung einer Proteinmasse, um Cystein-Peptidase-Inhibitoren hervorzubringen, wobei danach die Herstellung von Cystein-Peptidase-Inhibitoren und danach die Rückgewinnung eines chromatografischen Gels erfolgt, um Cystein-Peptidase-Inhibitoren hervorzubringen, **dadurch gekennzeichnet, dass** das Verfahren die folgenden Schritte umfasst:
a) die Bereitstellung einer Proteinmasse, um Cystein-Peptidase-Inhibitoren hervorzubringen, indem eine Proteinmasselösung einem sauren pH-Wert nahe bei pH 2 sowie einer hohen Temperatur nahe bei 90°C für eine Dauer von 10 bis 20 Minuten ausgesetzt wird, wobei die Proteinmasselösung danach für mehrere Stunden, vorzugsweise für 12 Stunden, eingefroren wird;
b) die Herstellung von Cystein-Peptidase-Inhibitoren durch zumindest eine Affinitätschromatografie, vorzugsweise auf einer Säule, gegen Träger mit gebundenem Papain oder Phycin oder einem anderen Derivat von pflanzlichen Cystein-Peptidasen unter Verwendung von aufgetauter Proteinmasse aus Schritt a) mit einer Aktivität von zumindest 5 IE/mg in dem Eluenten nach der ersten Elution, wobei die Rückgewinnung der Inhibitoren, welche an das Peptid gebunden sind, mit 20%igem Glycerol mit einem pH-Wert von 9,5 bis 12 und bei einer Temperatur von 8°C bis 55°C während mehrfacher Verwendung des chromatografischen Gels, oder bei einer Temperatur von 60°C bis 100°C unter einmaliger Verwendung des chromatografischen Gels durchgeführt wird.

2. Verfahren nach Anspruch 1, **dadurch gekennzeichnet, dass** eine zumindest zweifache Elution auf demselben chromatografischen Gel wie in Schritt b) durchgeführt wird.

3. Verfahren nach Anspruch 1 oder 2, **dadurch gekennzeichnet, dass** die Proteinmasse aus Eiern gewonnen wird, welche mit destilliertem Wasser verdünnt werden, vorzugsweise im Verhältnis 1:1 zwischen der Eiermasse und dem Wasser oder einem anderen biologischen Material, ausgewählt aus einer Gruppe umfassend pflanzliche Homogenate wie Kartoffeln, Sojabohnen, Reis, Misteln oder Säugetiergewebe wie Plazenten, chirurgisch entferntes Krebsgewebe sowie amniotische Flüssigkeit, Urin und ödemische Flüssigkeit.

4. Verfahren nach Anspruch 1, **dadurch gekennzeichnet, dass** die Herstellung der Cystein-Peptidase-Inhibitoren in Schritt b) das Zentrifugieren der aufgetauten Proteinmasse aus Schritt a), vorzugsweise für eine Dauer von mehr als 20 Minuten mit 30.000 Umdrehungen pro Minute, umfasst.

5. Verfahren nach Anspruch 4, **dadurch gekennzeichnet, dass** in Schritt b) zentrifugierter Überstand auf eine Säule geladen wird, vorzugsweise gepackt mit Sepharose 4B, welche zumindest zweifach an eine Cystein-Peptidase gebunden ist, vorzugsweise mit 0,05%igem Natriumsorbat, und anschließend gewaschen wird, vorzugsweise zunächst in 0,2 M NaCl und danach in destilliertem Wasser, bis in dem Filtrat kein biologisches Protein mehr nachweisbar ist.

6. Verfahren nach Anspruch 1, **dadurch gekennzeichnet, dass** das hervorgebrachte Eluat im Anschluss an die finale Elution in Schritt b) angesäuert wird, vorzugsweise auf einen pH-Wert von 2,0 und erhitzt wird, vorzugsweise auf 90°C für einen Zeitraum von 10 bis 20 Minuten, sowie abgekühlt und neutralisiert wird.

7. Verfahren nach Anspruch 1, **dadurch gekennzeichnet, dass** die Elutionen aus Schritt b) bis zum Punkt der elektrophoretischen Reinheit, vorzugsweise zwei, drei oder viermal auf aufeinanderfolgenden chromatografischen Gelsäulen mit immobilisierter Cystein-Peptidase durchgeführt werden, wodurch eine Aktivität von zumindest 5 IE/mg in dem Eluenten nach der ersten Elution aus der ersten Affinitätschromatografie hervorgebracht wird, wobei die Elutionen vorzugsweise mit einem pH-Wert von 11 und bei Raumtemperatur durchgeführt werden.

8. Verfahren nach Anspruch 1, **dadurch gekennzeichnet, dass** in Schritt b) im Anschluss an die Elution das chromatografische Gel mit dem immobilisierten Enzym durch Inkubation in einem Puffer reaktiviert wird, vorzugweise über einen Zeitraum von mindestens 12 Stunden bei 4°C in einem Phosphatpuffer mit einem pH-Wert von 6,5 und 2 mM Dithiothreitol, 2 mM Ethylendiamintetraessigsäure (EDTA) und 0,05% Mercaptoethanol, um das Enzym zu reaktivieren

## Revendications

1. Méthode de production d'inhibiteurs de cystéine peptidases de pureté électrophorétique à partir de matériaux biologiques englobant la préparation d'une masse protéinique pour produire des inhibiteurs de cystéine peptidases, après quoi la production des inhibiteurs de cystéine peptidases se produit et ensuite la récupération du gel de chromatographie pour produire des inhibiteurs de cystéine peptidases, **caractérisée en ce qu'**elle englobe :
a) la préparation d'une masse protéinique pour produire des inhibiteurs de cystéine peptidases en agissant sur une solution de la masse protéinique avec un pH acide proche de pH 2, ainsi qu'une température élevée proche de 90 C pendant 10 à 20 minutes, après quoi la solution de la masse protéinique est congelée pendant plusieurs heures, de préférence 12 ;
b) la production d'inhibiteurs de cystéine peptidases par l'intermédiaire d'au moins une chromatographie d'affinité, de préférence sur une colonne, contre des supports avec de la papaïne liée ou de la phycine liée ou un autre dérivé de cystéine peptidases végétales lié en utilisant la masse protéinique décongelée de l'étape a) avec une activité d'au moins 5 UI/mg dans l'éluant à la suite de la première élution, où la récupération des inhibiteurs liés au peptide est conduite avec 20 % de glycérol, avec un pH de 9,5 à 12 à une température de 8°C à 55°C durant un usage multiple du gel de chromatographie, ou à une température de 60°C à 100°C avec un usage unique du gel de chromatographie.

2. Méthode selon la revendication 1, **caractérisée en ce qu'**une élution est conduite au moins deux fois sur le même gel de chromatographie de l'étape b).

3. Méthode selon la revendication 1 ou 2, **caractérisée en ce que** la masse protéinique est obtenue à partir d'oeufs dilués avec de l'eau distillée, de préférence dans un rapport 1/1 de la masse des oeufs sur de l'eau ou d'un autre matériau biologique choisi dans un groupe contenant des homogénats de plantes telles que la pomme de terre, le soja, le riz, le gui ou des tissus de mammifères tels que le placenta, des tissus cancéreux prélevés durant une chirurgie ainsi que le liquide amniotique, l'urine, et le liquide d'oedème.

4. Méthode selon la revendication 1, **caractérisée en ce que** la production d'inhibiteurs de cystéine peptidases dans l'étape b) englobe la centrifugation de la masse protéinique décongelée de l'étape a), de préférence sur 20 minutes à 30 000 tr/min.

5. Méthode selon la revendication 4, **caractérisée en ce que** dans l'étape b), le surnageant centrifugé est chargé sur une colonne, de préférence remplie de Sepharose 4B lié à une cystéine peptidase, au moins deux fois, de préférence avec 0,05 % de sorbate de sodium et ensuite lavé, de préférence d'abord dans du NaCl 0,2 M et ensuite dans de l'eau distillée jusqu'à ce que l'on ne trouve plus de protéine biologique dans le filtrat.

6. Méthode selon la revendication 1, **caractérisée en ce que** l'éluat produit à la suite de l'élution finale dans l'étape b) est acidifié, de préférence à pH 2,0 et chauffé, de préférence à 90 °C pendant 10 à 20 minutes, refroidi et neutralisé.

7. Méthode selon la revendication 1, **caractérisée en ce que** les élutions de l'étape b) sont conduites jusqu'au point de pureté électrophorétique, de préférence deux, trois ou 4 fois sur des colonnes de gel de chromatographie subséquentes avec une cystéine peptidase immobilisée produisant une activité d'au moins 5 UI/mg dans l'éluant à la suite de la première élution à partir de la première chromatographie d'affinité, dans laquelle de préférence les élutions sont conduites à pH 11 et température ambiante.

8. Méthode selon la revendication 1, **caractérisée en ce que** dans l'étape b) à la suite de l'élution, le gel de chromatographie avec l'enzyme immobilisée est réactivé par l'intermédiaire d'une incubation dans un tampon, de préférence sur un minimum de 12 heures à 4°C dans du tampon phosphate avec un pH de 6,5 et 2 mM de dithiothréitol, 2 mM d'acide éthylène-diamine-tétra-acétique (EDTA) et 0,05% de mercaptoéthanol, afin de réactiver l'enzyme.
